# EUROPÄISCHE PATENTSCHRIFT

(11) **EP 2 813 485 B1**
(45) Veröffentlichungstag und Bekanntmachung des Hinweises auf die Patenterteilung: **18.05.2016**
(21) Anmeldenummer: 14160457.9
(22) Anmeldetag: 18.03.2014
(51) Int. Cl.: C07C 51/48

(54) **Verfahren zur Gewinnung von Bernsteinsaeure aus Fermentationsbrühen**
Method for the recovery of succinic acid from fermentation broths
Procédé de production d'acide succinique à partir de bouillons de fermentation

(30) Priorität: 21.03.2013 DE 102013004890
(43) Veröffentlichungstag der Anmeldung: 17.12.2014
(73) Patentinhaber: BASF SE, 67056 Ludwigshafen am Rhein (DE)
(72) Erfinder: Kiefer, Hans, 67435 Neustadt (DE)
(74) Vertreter: BASF IP Association

(56) Entgegenhaltungen:
- T.M Kurzrock: "Integrierte Reaktivextraktion zur Gewinnung von biotechnologisch hergestellter Bernsteinsäure (Dissertation)", , 14. Oktober 2010 (2010-10-14), Seiten FP-234, XP002729041, Technische Universität München Gefunden im Internet: URL:http://mediatum.ub.tum.de/doc/998307/9 98307.pdf [gefunden am 2014-10-09]

## Beschreibung

Bernsteinsäure wird in großem Umfang für verschiedene Anwendungen sowohl in der Lebensmittelindustrie als auch in der Pharmazie und der Kosmetik genutzt. Zunehmend dient Bernsteinsäure auch als Einsatzstoff bei der Herstellung von Polymeren. Bernsteinsäure kann sowohl auf chemischem Wege als auch durch Fermentation von Kohlenhydraten mittels verschiedener Mikroorganismen erzeugt werden.

Nachteilig beim fermentativen Herstellungsverfahren ist jedoch, dass die Bernsteinsäure hierbei nur als verdünnte wässrige Lösung (bis maximal 100 g/l) und auch nur in Form eines ihrer Salze, meist als Diammoniumsalz, anfällt. Zur Gewinnung der freien Säure muss aus diesem Salz erst noch durch Behandeln mit einer stärkeren Säure die Bernsteinsäure freigesetzt und isoliert werden.

Es hat daher auch schon Versuche gegeben, die Bernsteinsäure mittels Flüssig-flüssig-Extraktion aus der Fermentationsbrühe zu isolieren. Einen ausführlichen Stand der Technik über die Ergebnisse mit dieser Methode findet sich in der Dissertation von T. M. Kurzrock mit dem Titel "Integrierte Reaktivextraktion zur Gewinnung von biotechnologisch hergestellter Bernsteinsäure", eingereicht am 14. Oktober 2010 bei der TU München. In dieser Dissertation wird die Extraktion mit Hilfe "hochmolekularer aliphatischer Amine" favorisiert.

Aber auch hierbei wird die Bernsteinsäure salzartig an das Amin gebunden und muss daraus erst mit Hilfe einer stärkeren Säure freigesetzt werden. Außerdem weist der Autor darauf hin, dass die eingesetzten Amine toxisch gegenüber den zur Fermentation eingesetzten Mikroben seien, so dass bei deren unvollständiger Abtrennung mit einer "Limitierung des Zellwachstums und der Produktbildung zu rechnen ist". Eingang in die Technik hat daher diese Methode offensichtlich bisher nicht gefunden.

Es wurde jetzt ein verbessertes Verfahren zur Extraktion von Bernsteinsäure aus Fermentationsbrühen gefunden, das diese Nachteile nicht besitzt. Das erfindungsgemäße Verfahren zur Gewinnung von Bernsteinsäure aus Fermentationsbrühen ist dadurch gekennzeichnet, dass man die Fermentationsbrühe mit einem mit Wasser nicht mischbaren Nitril kontinuierlich im Gegenstrom extrahiert und danach die Bernsteinsäure aus der Extraktphase kontinuierlich im Gegenstrom mit heißem Wasser, das eine Temperatur im Bereich von 70 bis 100 °C aufweist, zurückextrahiert und durch Abkühlen der wässrigen Phase in kristalliner Form zur Abscheidung bringt.

Ein Nitril wird als mit Wasser nicht mischbar bezeichnet, wenn es bei einer Temperatur von 25 °C nicht in technisch relevanten Mengen in Wasser gelöst wird. Der Gehalt der Wasserphase an Nitril sollte bei einer Extraktion bei 25 °C vorzugsweise < 2 Gew.-%, besonders bevorzugt < 1 Gew.-% sein. Die Temperatur bei der Extraktion mit dem organischen Nitril kann frei gewählt werden und sollte vorzugsweise zwischen Umgebungstemperatur und Siedepunkt des Nitrils, jedoch unter 100 °C liegen. Vorzugsweise wird die Extraktion bei Umgebungstemperatur (20 bis 25 °C) durchgeführt.

Die Extraktionen können bei Normaldruck, vermindertem Druck oder erhöhtem Druck durchgeführt werden. Vorzugsweise werden sie bei Normaldruck durchgeführt.

Bei dem erfindungsgemäßen Verfahren werden vorzugsweise zunächst wie üblich die zur Fermentation eingesetzten Mikroben und andere Feststoffteilchen aus der Fermentationsbrühe entfernt, z. B. durch Filtration oder Zentrifugieren. Das Filtrat wird dann vorzugsweise mit einer anorganischen Säure, wie z. B. Schwefelsäure auf einen pH-Wert von 1 bis 4, vorzugsweise 1,5 bis 2,5, insbesondere 2, gebracht und anschließend mit einem Nitril extrahiert. Für diese Extraktion verwendet man vorzugsweise eine mehrstufige sogenannte Mixer-Settler-Apparatur oder eine mehrstufige Kolonne, wobei die zu extrahierende Fermentationsbrühe und das Extraktionsmittel jeweils im Gegenstrom geführt werden. Als Extraktionsmittel verwendet man erfindungsgemäß mit Wasser nicht mischbare organische Nitrile, vorzugsweise solche der Formel RCN, wobei es sich bei dem Rest R um ein Alkyl-, Cycloalkyl- oder Aryl-Rest handelt, welcher auch zusätzliche andere Substituenten tragen kann. Der Alkylrest weist vorzugsweise 3 bis 12 C-Atome auf, der Cycloalkyl- und Arylrest vorzugsweise 6 bis 12 C-Atome. Besonders bevorzugt sind Butyronitril und Benzonitril.

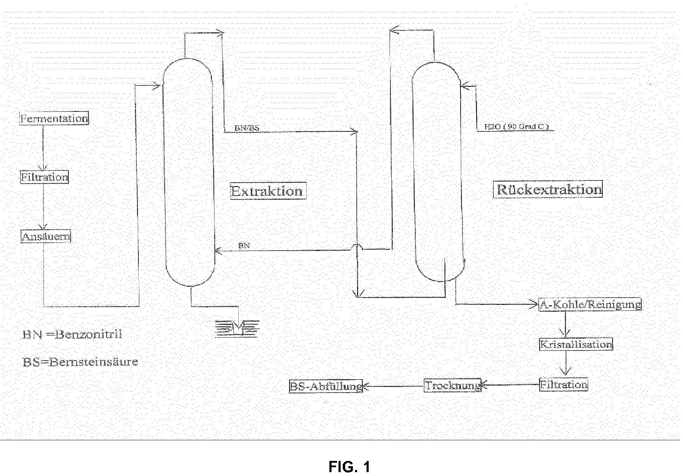

Aus der so gewonnenen Nitril-Phase wird danach die Bernsteinsäure mit heißem Wasser im Gegenstromverfahren vorzugsweise in einer mehrstufigen Mixer-Settler-Apparatur oder einer mehrstufigen Kolonne in die wässrige Phase zurückextrahiert. Man verwendet hierzu bevorzugt 85 bis 95 °C heißes Wasser. An die Rückextraktion kann sich ein Reinigungsschritt, z. B. durch Inkontaktbringen mit Aktivkohle, anschließen. Durch Abkühlen der wässrigen Phase auf vorzugsweise 5 bis 30 °C, besonders bevorzugt 10 bis 20 °C scheidet sich die Bernsteinsäure als praktisch farblose Kristalle ab. Diese lassen sich beispielsweise durch Filtration oder Zentrifugieren von der Mutterlauge abtrennen. Die noch geringe Mengen Bernsteinsäure enthaltende Mutterlauge kann zur Vermeidung von Produktverlusten erneut für die Extraktion der Bernsteinsäure aus der Nitril-Phase verwendet werden. Die technische Durchführung des neuen Verfahrens wird durch die Prinzipskizze in der Figur 1 näher erläutert.

## Patentansprüche

1. Verfahren zur Gewinnung von Bernsteinsäure aus Fermentationsbrühen, **dadurch gekennzeichnet, dass** man die Fermentationsbrühe mit einem mit Wasser nicht mischbaren Nitril kontinuierlich im Gegenstrom extrahiert und danach die Bernsteinsäure aus der Extraktphase kontinuierlich im Gegenstrom mit heißem Wasser, das eine Temperatur im Bereich von 70 bis 100 °C aufweist, zurückextrahiert und durch Abkühlen der wässrigen Phase in kristalliner Form zur Abscheidung bringt.

2. Verfahren nach Anspruch 1, **dadurch gekennzeichnet, dass** für die beiden Extraktionsschritte jeweils mehrstufige Kolonnen nach dem Gegenstromprinzip eingesetzt werden.

3. Verfahren nach Anspruch 1 oder 2, **dadurch gekennzeichnet, dass** das mit Wasser nicht mischbare Nitril die allgemeine Formel RCN hat, in der der Rest R die Bedeutung eines gegebenenfalls substituierten aliphatischen, cycloaliphatischen oder eines aromatischen Restes besitzt.

4. Verfahren nach Anspruch 3, **dadurch gekennzeichnet, dass** als Nitril der Formel RCN Butyronitril oder Benzonitril zur Extraktion eingesetzt werden.

5. Verfahren nach einem der Ansprüche 1 bis 4, **dadurch gekennzeichnet, dass** das zur Rückextraktion eingesetzte Wasser eine Temperatur im Bereich von 85 bis 95 °C hat.

6. Verfahren nach einem der Ansprüche 1 bis 5, **dadurch gekennzeichnet, dass** aus der Fermentationsbrühe vor den Extraktionen die zur Fermentation eingesetzten Mikroben und andere Feststoffteilchen entfernt werden.

7. Verfahren nach Anspruch 6, **dadurch gekennzeichnet, dass** nachfolgend, aber vor den Extraktionen die Fermentationsbrühe auf einen pH-Wert von 1 bis 4 gebracht wird.

## Claims

1. A method for the recovery of succinic acid from fermentation broths, wherein the fermentation broth is continuously extracted in counterflow with a water-immiscible nitrile and thereafter the succinic acid is continuously back-extracted from the extract phase in counterflow with hot water that has a temperature in the range from 70 to 100°C, and, by cooling the aqueous phase, is brought to separation in crystalline form.

2. The method according to claim 1, wherein, for the two extraction steps, in each case multistage columns are used according to the counterflow principle.

3. The method according to claim 1 or 2, wherein the water-immiscible nitrile has the general formula RCN, in which the radical R has the meaning of an optionally substituted aliphatic, cycloaliphatic, or an aromatic radical.

4. The method according to claim 3, wherein the nitrile of the formula RCN used for the extraction is butyronitrile or benzonitrile.

5. The method according to any one of claims 1 to 4, wherein the water used for the back extraction has a temperature in the range of from 85 to 95°C.

6. The method according to any one of claims 1 to 5, wherein, before the extractions, the microbes used for the fermentation and other solids particles are removed from the fermentation broth.

7. The method according to claim 6, wherein subsequently, but before the extractions, the fermentation broth is brought to a pH from 1 to 4.

## Revendications

1. Procédé de production d'acide succinique à partir de bouillons de fermentation, **caractérisé en ce que** l'on extrait le bouillon de fermentation à contre-courant en continu dans un nitrile non miscible dans l'eau et l'on réextrait ensuite l'acide succinique de la phase d'extraction en continu à contre-courant avec de l'eau chaude à une température dans une plage de 70 à 100°C et on l'amène par refroidissement de la phase aqueuse à une forme cristalline en vue de sa séparation.

2. Procédé selon la revendication 1, **caractérisé en ce que** pour les deux étapes d'extraction, on utilise à chaque fois des colonnes multiétagées selon le principe des contre-courants.

3. Procédé selon la revendication 1 ou 2, **caractérisé en ce que** le nitrile non miscible dans l'eau présente la formule générale RCN dans laquelle le radical R représente un radical aliphatique, cycloaliphatique ou aromatique éventuellement substitué.

4. Procédé selon la revendication 3, **caractérisé en ce que** l'on utilise en tant que nitrile de formule RCN du butyronitrile ou du benzonitrile en vue de l'extraction.

5. Procédé selon l'une quelconque des revendications 1 à 4, **caractérisé en ce que** l'eau utilisée pour la réextraction est à une température dans une plage de 85 à 95°C.

6. Procédé selon l'une quelconque des revendications 1 à 5, **caractérisé en ce que** l'on élimine du bouillon de fermentation avant les extractions les microbes utilisés pour la fermentation et d'autres particules solides.

7. Procédé selon la revendication 6, **caractérisé en ce qu'**ensuite, mais avant les extractions, le bouillon de fermentation est amené à un pH de 1 à 4.
